# EUROPÄISCHE PATENTSCHRIFT

(11) **EP 0 358 595 B1**
(45) Veröffentlichungstag und Bekanntmachung des Hinweises auf die Patenterteilung: **11.05.1994**
(21) Anmeldenummer: 89730160.2
(22) Anmeldetag: 12.07.1989
(51) Int. Cl.: C07D 413/14, A61K 31/415, C07D 413/04, C07D 417/04, C07D 417/14, C07D 403/04, C07D 401/04

(54) **N-Hetaryl-imidazolderivate**
N-hetaryl imidazole derivatives
Dérivés N-hétaryl d'imidazol

(30) Priorität: 15.07.1988 DE 3824658
(43) Veröffentlichungstag der Anmeldung: 14.03.1990
(73) Patentinhaber: SCHERING AKTIENGESELLSCHAFT, 13342 Berlin (DE)
(72) Erfinder: Rohde, Ralph, Dr., D-1000 Berlin 45 (DE); Biere, Helmut, Dr., D-1000 Berlin 28 (DE); Schmiechen, Ralph, Dr., D-1000 Berlin 42 (DE); Andrews, John Stuart, D-1000 Berlin 30 (DE); Stephens, David Norman, Dr., D-1000 Berlin 33 (DE)

(56) Entgegenhaltungen:
- EP-A- 0 091 596
- EP-A- 0 125 029
- EP-A- 0 144 098
- EP-A- 0 200 299
- EP-A- 0 205 067
- EP-A- 0 323 799
- AT-A- 374 198
- GB-A- 2 111 996
- US-A- 4 746 669
- US-A- 4 756 744

## Beschreibung

Die Erfindung betrifft neue ZN-aktive Imidazolderivate der allgemeinen Formel I 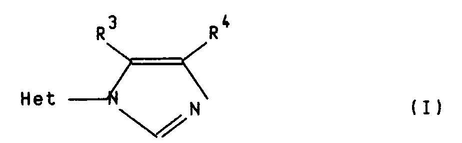
worin
- Het: ein mono- oder bicyclischer 5-10 gliedriger Heteroaromat mit 1-3 Schwefel-, Sauerstoff- und/oder Stickstoffatomen bedeutet, der ein bis zweifach substituiert sein kann mit Halogen, einem geradkettigen verzweigten, cyclischen, gesättigten oder ungesättigten C₁₋₆-Kohlenwasserstoffrest, Nitro, Cyano, Thiocyanat, Azid, C₁₋₅-Alkanoyl, C₁₋₄-Alkoxycarbonyl, Carboxyl, einer gegebenenfalls mit C₁₋₄-Alkyl oder C₂₋₅-Acyl mono- oder disubstituierte Amino- oder Amidgruppe, C₁₋₄-Alkoxy, C₁₋₆-Alkylthioalkyl oder einem Sulfoxid-, Sulfonyl- oder Sulfonylaminorest,
- R³: Wasserstoff, eine gerade oder verzweigte C₁₋₆-Alkylgruppe oder eine C₁₋₆-Alkyoxyalkylgruppe und
- R⁴: -COOR⁵, -CONR⁶R⁷, -CN,

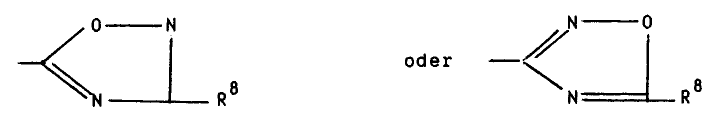

bedeuten,
mit R⁵ in der Bedeutung von Wasserstoff, einer geraden oder verzweigten C₁₋₆-Alkylgruppe, R⁶ und R⁷ sind gleich oder verschieden und stellen Wasserstoff oder eine gerade, verzweigte oder cyclische Alkylgruppe mit bis zu 7 Kohlenstoffatomen dar oder bilden gemeinsam mit dem Stickstoffatom einen gegebenenfalls mit ein bis zwei C₁₋₄-Alkylgruppen substituierten Pyrrolidin-, Piperidin-, Morpholin-, Piperazin- oder Thiomorpholin-Ring bedeuten und R⁸ Wasserstoff oder eine gerade, verzweigte oder cyclische Alkylgruppe mit bis zu 7 Kohlenstoffatomen bedeutet.

Der mono- oder bicyclische Heteroaromat Het ist bevorzugt 5-10-gliedrig und enthält 1-3 Heteroatome wie Schwefel, Sauerstoff und/oder Stickstoff. Beispielsweise seien die folgenden heteroaromatischen Reste genannt: Thienyl, Furyl, Pyrrolyl, Pyranyl, Imidazolyl, Pyrazolyl, Pyridyl, Pyrimidinyl, Pyrazinyl, Pyridazinyl, Oxazolyl, Isoxazolyl, Thiazolyl, Isothiazolyl, Thiadiazolyl, Triazolyl, Triazinyl, Benzothiazolyl, Benzimidazolyl, Indolyl, Benzofuranyl, Benzothienyl.

Als bevorzugt zu betrachten sind 5-6-gliedrige Heteroaromaten, die 1-3 Heteroatome enthalten können und gegebenenfalls einen ankondensierten Benzolring haben.

Der Heteroaromat kann an jeder beliebigen Stelle substituiert sein mit 1 - 2 Substituenten R¹ und, falls ein ankondensierter Benzolring vorhanden ist, kann dieser in o-, m- oder p-Stellung ein- bis zweifach substituiert sein mit einem Substituenten R².

R¹ und R² können jeweils Halogen wie Fluor, Chlor, Brom und Jod, einen geradkettigen, verzweigten, cyclischen, gesättigten oder ungesättigten C₁₋₆-Kohlenwasserstoffrest, Nitro, Cyano, Thiocyanat, Azid, C₁₋₅-Alkanoyl, C₁₋₄-Alkoxycarbonyl, Carboxyl, eine gegebenenfalls mit C₁₋₄-Alkyl oder C₂₋₅-Acyl mono- oder disubstituierte Amino- oder Amidgruppe, C₁₋₄-Alkoxy, C₁₋₆-Alkylthioalkyl oder einen Sulfoxid-, Sulfonyl- oder Sulfonylaminorest bedeuten.

Als bevorzugte Substituenten R¹ und R² sind die folgenden Reste zu betrachten: Halogen, geradkettiges oder verzweigtes C₁₋₆-Alkyl, C₁₋₄-Alkoxy, Nitro oder gegebenenfalls substituiertes Amin.

Unter einem Kohlenwasserstoffrest sind insbesondere gesättigte oder ungesättigte, geradkettige oder verzweigte C₁₋₆-Alkylreste und C₃₋₆-Cycloalkylreste zu verstehen.

Als gesättigte, geradkettige oder verzweigte Alkylreste sind jeweils die niederen Alkylreste wie Methyl, Ethyl, Propyl, i-Propyl, Butyl, sek. Butyl, Isobutyl, tert. Butyl sowie Pentyl, Hexyl, 2-Methylbutyl, 2,2-Dimethylpropyl gemeint.

Als ungesättigte Alkylgruppen seien als bevorzugt die folgenden Alkenyl-und Alkinylreste genannt: 1-Propenyl, 2-Propenyl, 3-Methyl-2-propenyl, 2-Propinyl. Als Cycloalkyle seien beispielsweise Cyclopropyl, Cyclobutyl, Cyclopentyl, Cychlohexyl und Cycloheptyl genannt.

Bilden R⁶, R⁷ gemeinsam mit dem Stickstoffatom einen gegebenenfalls ein weiteres Heteroatom enthaltenden gesättigten heterocyclischen Fünf- oder Sechsring, so stellt dieser beispielsweise Pyrrolidin, Piperidin, Morpholin, Piperazin oder Thiomorpholin dar und kann gegebenenfalls mit ein bis zwei C₁₋₄-Alkylgruppen substituiert sein wie beispielsweise 2,6-Dimethylmorpholin oder N-Methylpiperazin.

Als bevorzugte Ausführungsform für R³ in Bedeutung Alkoxyalkyl ist C₁₋₄-Alkoxy-C₁₋₂-Alkyl, insbesondere C₁₋₄-Alkoxymethyl, und in der Bedeutung Alkyl gerade oder verzweigte Alkylgruppen mit 1-4 Kohlenstoffatomen anzusehen. Als bevorzugte Ausführungsform für R⁴ ist COOR⁵, 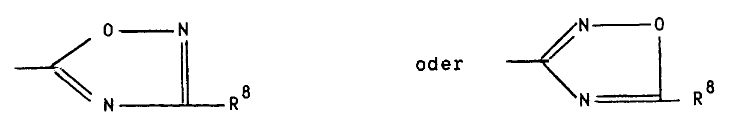
zu betrachten, wobei R⁵ und R⁸ als Alkylreste und R⁸ als Cycloalkylrest insbesondere bis zu 4 Kohlenstoffatome haben. Insbesondere bevorzugt ist R⁴ = COOR⁵ und 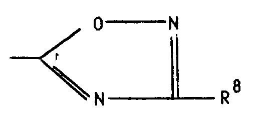
Die erfindungsgemäßen Verbindungen erscheinen aufgrund ihrer biologischen Wirksamkeit als Psychopharmaka für die Humanmedizin geeignet. Da die neuen Verbindungen sehr gut anxiolytisch wirksam sind und weder sedierende noch muskelrelaxierende Nebenwirkungen autreten, können sie insbesondere als Anxiolytika verwendet werden. Sie können zu psychopharmazeutischen Präparationen, zum Beispiel für die orale und parenterale Anwendung, formuliert werden.

Als Formulierungshilfsstoffe sind physiologisch verträgliche organische und anorganische Trägersubstanzen geeignet, die gegenüber den erfindungsgemäßen Verbindungen inert sind.

Als Trägersubstanzen seien beispielsweise genannt Wasser, Salzlösungen, Alkohole, Polyäthylenglykole, polyhydroxyethoxyliertes Rizinusöl, Gelatine, Lactose , Amylose, Magnesiumstearat, Talkum, Kieselsäure, Fettsäure-mono- und -diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermitteln, Konservierungsstoffen, Stabilisatoren, Netzmitteln, Emulgatoren, Puffermitteln und Farbstoffen, versetzt werden.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet. Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder einem Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die erfindungsgemäßen Verbindungen werden in einer Dosiseinheit von 0,05 bis 10 mg aktiver Substanz in einem physiologisch verträglichen Träger eingebracht.

Die erfindungsgemäßen Verbindungen werden in einer Dosis von 0,1 bis 300 mg/Tag vorzugsweise 1 - 30 mg/Tag, angewendet.

Die Herstellung der erfindungsgemäßen Verbindungen der allgemeinen Formel I erfolgt dadurch, daß man
a) ein Amin der allgemeinen Formel II

   Het―NH₂ (II),

   worin
   Het die in Formel I angegebene Bedeutung hat, mit einem 2-Azadien der allgemeinen Formel III 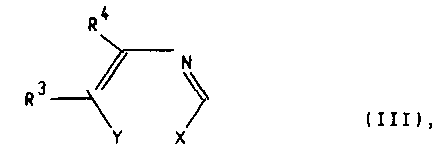 worin
   R³ und R⁴ die in Formel I angegebene Bedeutung haben und
   X und Y Fluchtgruppen darstellen,
   in Gegenwart von einem Säureüberschuß bei Temperaturen von 0 bis 150°C umsetzt oder
b) ein Imidazolderivat der allgemeinen Formel IV 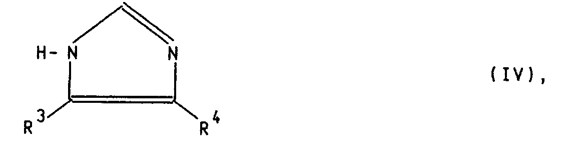 worin
   R³ und R⁴ die oben genannte Bedeutung haben, mit einem Heteroaromaten der allgemeinen Formel Het-Z, worin Z eine Fluchtgruppe darstellt, N-aryliert und gegebenenfalls auftretende Isomeren trennt und anschließend gegebenenfalls in den nach a) oder b) erhaltenen Verbindungen eine im Molekül vorhandene Estergruppe umestert oder verseift, eine freie Carboxylgruppe gegebenenfallls verestert, amidiert oder mit einem Amidoxim der Formel R⁸-C(=NOH)NH₂ zum 5-Oxadiazolylderivat umsetzt und gegebenenfalls eine im Molekül vorhandene Nitrilgruppe zur Carbonylamid- oder Carboxylgruppe hydrolisiert oder über die Iminoestergruppe in die Estergruppe (COOR⁵) oder mit Hydroxylamin über das Amidoxim und anschließend mit einer Alkancarbonsäure der Formel R⁸-COOH oder einem aktivierten Derivat der Säure in das 3-Oxadiazolylderivat überführt und gegebenenfalls eine Nitro-Gruppe zur Aminogruppe reduziert und diese anschließend gegebenenfalls alkyliert, acyliert, sulfonyliert oder über eine Diazotierung gegen Halogen, Azid, Cyano oder Thiocyanat austauscht.

Die erfindungsgemäße Umsetzung von Aminen der Formel II mit 2-Azadienen der Formel III zu den Imidazolderivaten der Formel I erfolgt in Gegenwart von Säuren bei Temperaturen von 0 bis 150 °C. Die Fluchtgruppen X und Y können gleich oder verschieden sein; insbesondere geeignet sind C₁₋₃-Dialkylamine, wie Dimethyl-, Diethyl-und Dipropylamin, und cyclische Amine, wie Pyrrolidin.

Die Umsetzung wird beispielsweise so ausgeführt, daß das Aminderivat und das Azadien in einer organischen Säure, wie zum Beispiel Ameisensäure, Essigsäure, Propionsäure oder Trifluoressigsäure, zunächst bei Raumtemperatur gerührt und dann bis zur Siedetemperatur des Reaktionsgemisches (bis etwa 120 °C) erhitzt wird.

Die Säure kann gleichzeitig als Reaktionsmittel und auch als Lösungsmittel dienen. Es können aber auch Lösungsmittel wie zum Beispiel Alkohole, Ether, Ketone, Ester, wie Ethylacetat, Kohlenwasserstoffe, wie Toluol, oder Halogenkohlenwasserstoffe, wie Tetrachlorkohlenstoff, zugesetzt werden.

Die Menge der Säure kann in weiten Grenzen variiert werden, sie wird jedoch im Überschuß angewendet. Vorzugsweise wird ein 3 - 10facher Säureüberschuß, bezogen auf das Amin und das Azadien, gewählt.

Die Molverhältnisse von Amin und Azadien sind für den Erfolg der Umsetzung nicht kritisch. Im allgemeinen wird man etwa gleiche molare Mengen der Reaktionspartner einsetzen, wobei Mengenverhältnisse von 1 Mol Amin und 1-3 Mol Azadien bevorzugt sind. Die erfindungsgemäße Umsetzung kann grundsätzlich auch in den oben angegebenen Lösungsmitteln mit katalytischen Mengen von Mineralsäuren, wie Schwefelsäure, Salzsäure, Perchlorsäure oder organischen Säuren wie p-Toluolsulfonsäure und Trifluoressigsäure, durchgeführt werden.

Der Vorteil des erfindungsgemäßen Verfahrens nach der Methode a) liegt in der chemoselektiven Synthese von Imidazolderivaten unter Bildung nur eines Isomeren in einer einzigen Verfahrensstufe.

Die N-Arylierung der Imidazolderivate der allgemeinen Formel IV kann beispielsweise nach der von N.W. Gilman et al. J. Heterocycl. Chem. 14, 1157 (1977) beschriebenen Methode erfolgen. Als Fluchtgruppe Z kommen Halogene, insbesondere Fluor und Jod, in Betracht. Die Arylierung nach der Methode b) wird in Gegenwart von Basen wie Alkalihydroxid, Alkalihydrid gegebenenfalls in Gegenwart von Phasentransferkatalysatoren, Butyllithium oder Lithiumdiisopropylamid, vorzugsweise mit Alkalihydrid, durchgeführt.

Für die Umsetzung sind Temperaturen von - 78 °C bis 100 °C, vorzugsweise von 0 °C bis 50 °C geeignet. Als Lösungsmittel für die Arylierung kommen aprotische polare Lösungsmittel in Betracht, beispielsweise aliphatische und cyclische Ether wie Diethylether, Tetrahydrofuran, u.a. und Dimethylformamid.

Für die sich gegebenenfalls anschließende Umesterung sind alle gebräuchlichen Methoden geeignet. Beispielsweise genannt seien die Umsetzung des Carbonsäureesters mit dem entsprechenden Alkohol in Gegenwart des Alkoholats oder mit dem entsprechenden Alkohol mit Titan-tetraalkoholat oder mit dem Alkohol in Gegenwart einer Säure . Die Umesterung wird bei Temperaturen von etwa 0 bis 120 °C durchgeführt.

Die sich gegebenenfalls anschließende Verseifung der Estergruppe erfolgt zweckmäßigerweise alkalisch, wobei der Ester in verdünnter wäßriger oder alkoholischer Alkalilauge, wie Kalium- oder Natriumhydroxid, bis zur Rückflußtemperatur erhitzt wird.

Die Veresterung der Carboxylgruppe geschieht in an sich bekannter Weise mit dem entsprechenden Alkohol in Säure oder in Gegenwart eines aktivierten Säurederivats. Als aktivierte Säurederivate kommen zum Beispiel Säurechlorid, -imidazolid oder -anhydrid infrage.

Zur Amidierung wird die Imidazol-4-carbonsäure oder der entsprechende Ester mit Hilfe von N,N'-Carbonyldiimidazol oder Dicyclohexylcarbodiimid mit einem primären oder sekundärem Amin der allgemeinen Formel 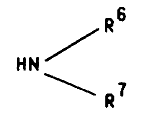
umgesetzt.

Die Umsetzung kann auch in an sich bekannter Weise über aktivierte Säurederivate wie über das Anhydrid oder das gemischte Anhydrid mit Chlorameisensäureester erfolgen. Üblicherweise wird die Amidierung in einem aprotischen Lösungsmittel wie Dimethylformamid, Tetrahydrofuran, Toluol oder Methylenchlorid bei Temperaturen von etwa 0 bis 100 °C vorgenommen.

Zur Einführung des 5-Oxadiazolylrestes kann auch die Imidazol-4-carbonsäure mit einem Amidoxim der Formel R⁸-C(=NOH)NH₂, in der R⁸ die in Formel I angegebene Bedeutung hat, in einem inerten Lösungsmittel bei Raumtemperatur bis zur Siedetemperatur des Reaktionsgemisches zur Kondensation gebracht werden. Als inerte Lösungsmittel sind beispielsweise Toluol und Dimethylformamid geeignet. Zweckmäßigerweise wird die freie Carbonsäure vor der Kondensationsreaktion in geeigneter Weise aktiviert. Hierzu kann die freie Säure in das gemischte Anhydrid, in den aktivierten Ester oder in das Chlorid überführt werden. Gut bewährt hat sich eine Aktivierung mit Imidazol/Thionylchlorid in einem aprotischen Lösungsmittel wie Dioxan, Tetrahydrofuran, Dimethylformamid oder N-Methylpyrrolidon bei Temperaturen zwischen 0 und 50 °C.

Die sich gegebenenfalls anschließende Abwandlung der Nitrilgruppe kann nach bekannten Methoden durchgeführt werden. Beispielsweise kann die Nitrilgruppe durch saure oder alkalische Hydrolyse in die Carbonylamid- oder Carboxylgruppe oder mit dem entsprechenden Alkohol unter Zugabe von Chlorwasserstoffgas über die Iminoestergruppe in die Estergruppe überführt werden.

Zur Einführung des 3-Oxadiazolylrestes wird das Imidazol-4-carbonitril in an sich bekannter Weise mit Hydroxylamin zum Amidoxim umgesetzt und anschließend mit einer Alkancarbonsäure der Formel R⁸-COOH, in der R⁸ die in Formel I angegebene Bedeutung hat, oder einem aktivierten Derivat der Säure in einem inerten Lösungsmittel kondensiert. Die Kondensation wird in der gleichen Weise wie bei der 5-Oxadiazolylverbindung durchgeführt.

Die Reduktion der Nitrogruppe zur Aminogruppe kann beispielsweise katalytisch erfolgen, indem man unter Normaldruck oder H₂-Druck in polaren Lösungsmitteln bei Raumtemperatur hydriert. Als Katalysator kann Palladium auf einem Träger wie Kohle oder Platin in feinverteilter Form verwendet werden; bei Verbindungen mit Halogen verwendet man als Katalysator vorzugsweise Raney-Nickel. Für die Reduktion geeignete polare Lösungsmittel sind beispielsweise Alkohole oder Ether wie Methanol, Ethanol, Diethylether, Tetrahydrofuran oder deren Mischungen .

Die Einführung der Cyanogruppe kann mit Hilfe der Sandmeyer-Reaktion erfolgen; beispielsweise kann man die aus den Aminoverbindungen mit Nitriten intermediär gebildeten Diazoniumsalze mit Alkalicyaniden in Gegenwart von Cu-I-cyanid umsetzen.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Cu(I)chlorid oder Cu(I)bromid in Gegenwart der entsprechenden Säure Salzsäure oder Bromwasserstoffsäure umsetzt oder mit Kaliumiodid umsetzt.

Die Einführung von Fluor gelingt beispielsweise durch Balz Schiemann-Reaktion des Diazoniumtetrafluorborates.

Die Einführung der Azido- oder Thiocyanatgruppe kann ebenfalls über Sandmeyer-Reaktion des Diazoniumsalzes mit Alkaliazid oder Alkalithiocyanat erfolgen.

Wird eine Alkylierung, Acylierung oder Sulfonylierung der Aminogruppe gewünscht, so kann nach üblichen Methoden beispielsweise mit Alkyl-, Acyl-oder Sulfonylhalogeniden alkyliert, acyliert oder sulfonyliert werden.

### Ausgangsstoffe:

Die als Ausgangsstoffe benutzten Aminoheterocyclen der allgemeinen Formel II und die Azadiene der allgemeinen Formel III sind überwiegend bekannt oder können nach bekannten Methoden hergestellt werden.

2-Azadiene sind zum Beispiel in Liebigs Ann.Chem. 1980, 344 , DE-OS 29 19 891 und in Liebigs Ann.Chem. 1986, 1749 beschrieben.

Die Herstellung der in den Beispielen eingesetzten 2-Azadiene wird anhand der folgenden Beispiele erläutert.

### Azadien 1:

### 1,4-Bis(dimethylamino)-2-aza-1,3-butadien-3-carbonsäure-ethylester :

Die Synthese erfolgt gemäß Liebigs Ann.Chem. 1980, 344.

### Azadien 2:

### 1,4-Bis(dimethylamino)-3-(3-ethyl-1,2,4-oxadiazol-5-yl)-2-aza-1,3-butadien :

Die Synthese erfolgt gemäß Liebigs Ann.Chem. 1986,1749.

### Azadien 3:

a) 3-Ethyl-5-(N-dimethylaminomethylenaminomethyl)-1,2,4-oxadiazol :
   Eine Mischung aus 26 g 5-Aminomethyl-3-ethyl-1,2,4-oxadiazol und 30 ml Dimethylformamid-dimethylacetal wird 6 1/2 Stunden auf 80 °C (Badtemperatur) erhitzt. Anschließend werden 16 ml Methanol abdestilliert und das gebildete Produkt wird durch Kugelrohrdestillation gereinigt. Man erhält 27,9 g (74,8 %) öl mit dem Siedepunkt 130 - 150 °C (0,05 Torr/0,06 mbar); n_{D}²⁰: 1,4924.
b) 1-Dimethylamino-3-(3-ethyl-1,2,4-oxadiazol-5-yl)-4-(1-pyrrolidinyl)-2-azapenta-1,3-dien (E.Z-Gemisch) :
   13,6 g des unter a erhaltenen Produktes, 15,0 g Dimethylacetamid-dimethylacetal und 8,0 g Pyrrolidin werden 21 Stunden unter Stickstoff auf 80 °C (Badtemperatur) erhitzt. Anschließend wird der gebildete Alkohol abdestilliert und das Reaktionsprodukt durch Kugelrohrdestillation gereinigt. Man erhält 15,4 g einer Fraktion, die bei 215-230°C (0,04 Torr/0,05mb.)destilliert. Durch Umkristallisation aus n-Hexan gewinnt man 9,5 g (45,7 %) Azadien 3 mit einem Schmelzpunkt von 59 - 62 °C.

### Azadien 4:

a) Methoxyessigsaure-dimethylamid-dimethylacetal:
   Zu 53,4 g Trimethyloxonium-tetrafluoroborat werden unter Kühlung 42,2 g Methoxyessigsäure-dimethylamid in drei Portionen gegeben. Das Reaktionsgemisch wird anschließend 2 Stunden bei Raumtemperatur gerührt und danach über Nacht stehengelassen. Nach dem Lösen in 40 ml Dichlormethan wird das gebildete Salz langsam zu einer Lösung von Natriummethoxid in Methanol ( hergestellt durch Auflösen von 10,4 g Natrium in 225 ml Methanol) gegeben. Danach wird noch 2 Stunden bei Raumtemperatur gerührt. Zur Aufarbeitung wird der gebildete Niederschlag abgesaugt und mit wenig Ethanol ausgewaschen. Das Filtrat bildet nach dem Abdestillieren der Lösungsmittel 2 Phasen. Durch Kugelrohrdestillation erhält man aus der oberen Phase 21,8 g (36 %) des gewünschten Produktes mit einem Siedepunkt von 54 - 57 °C (14 Torr/18,7 mbar).; n_{D}²⁰: 1,4204.
b) 1-Dimethylamino-3-(3-ethyl-1,2,4-oxadiazol-5-yl )-5-methoxy-4-(1-pyrrolidinyl)-2-azapenta-1,3-dien (E,Z-Gemisch) :
   8,8 g des unter a dargestellten Produktes werden mit 6,6 g 3-Ethyl-5-(N-dimethylaminomethylenaminomethyl)-1,2,4-oxadiazol und 4,5 ml Pyrrolidin analog der Herstellung von Azadien 3 umgesetzt. Durch Kugelrohrdestillation erhält man 11,8 g (quant.) Azadien 4 mit dem Siedepunkt 200 - 240 °C (0,05 Torr/0,06 mbar).

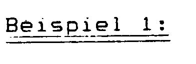

a) 1-(2-Thiazolyl)-imidazol-4-carbonsäure-ethylester:
   Eine Lösung aus 1,94 g Azadien 1 in 7 ml Eisessig wird unter Kühlung mit 700 mg 2-Aminothiazol versetzt und anschließend 16 Stunden bei Raumtemperatur unter Stickstoff gerührt. Danach wird 3 Stunden auf 100 °C (Badtemperatur) erhitzt. Zur Aufarbeitung wird das Reaktionsgemisch unter Kühlung mit Kaliumcarbonatlösung versetzt, das gebildete Kristallisat abgesaugt, mit Wasser nachgewaschen und getrocknet. Durch Umkristallisation aus Essigester erhält man 986 mg (63 %) der Titelverbindung mit einem Schmelzpunkt von 142 - 143 °C.
   Analog Beispiel 1a) werden erhalten:
b) 4-(3-Ethyl-1,2,4-oxadiazol-5-yl)-1-(2-thiazolyl)-imidazol:
   durch Umsetzung von Azadien 2 mit 2-Aminothiazol; Schmelzpunkt 147 - 148 °C (Ethanol).
c) 1-(5-Methyl-3-isoxazolyl)-imidazol-4-carbonsäure-ethylester:
   durch Umsetzung von Azadien 1 mit 3-Amino-5-methylisoxazol; Schmelzpunkt 142 - 143 °C (Ethanol).
d) 1-(5-Methyl-1,3,4-thiadiazol-2-yl)-imidazol-4-carbonsaure-ethylester:
   durch Umsetzung von Azadien 1 mit 2-Amino-5-methyl-1,3,4-thiadiazol; Schmelzpunkt 121 - 122 °C (Ethanol).
e) 1-(1H-1,2,4-Triazol-3-yl)-imidazol-4-carbonsäure-ethylester:
   durch Umsetzung von Azadien 1 mit 3-Amino-1,2,4-triazol; Schmelzpunkt 220 - 222 °C (Ethanol).
f) 4-(3-Ethyl-1,2,4-oxadiazol-5-yl)-1-(1H-1,2,4-triazol-3-yl)-imidazol:
   durch Umsetzung von Azadien 2 mit 3-Amino-1,2,4-triazol; Schmelzpunkt 242 - 243 °C (Ethanol).

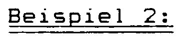

a) 1-(2-Benzothiazolyl)-imidazol-4-carbonsäure-ethylester:
   1,4 g Azadien 1 werden mit 751 mg 2-Aminobenzothiazol in 5 ml Eisessig in der unter Beispiel 1a) angegebenen Weise umgesetzt. Nach Aufarbeitung und Umkristallisation aus Ethanol erhält man 627 mg (45,9 %) der Titelverbindung mit einem Schmelzpunkt von 131 - 133 °C. Analog Beispiel 2a) werden erhalten:
b) 1-(2-Benzimidazolyl)-imidazol-4-carbonsäure-ethylester:
   durch Umsetzung von Azadien 1 mit 2-Aminobenz-imidazol; Schmelzpunkt 252 - 253 °C (Ethanol).
c) 4-(3-Ethyl-1,2,4-oxadiazol-5-yl)-1-(3-indolyl)-imidazol:
   durch Umsetzung von Azadien 2 mit 3-Aminoindol; Schmelpunkt 198 - 199 °C (Acetonitril).

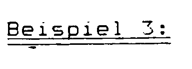

a) 1-(3-Pyridyl)-imidazol-4-carbonsäure-ethylester:
   5,6 g Azadien 1 werden mit 1,9 g 3-Aminopyridin in 20 ml Eisessig in der unter Beispiel 1a) beschriebenen Weise umgesetzt. Nach Aufarbeitung und Umkristallisation aus Toluol erhält man 2,39 g (55 %) der Titelverbindung mit einem Schmelzpunkt von 102 - 103 °C
   Analog Beispiel 3a) werden erhalten:
b) 1-(2-Pyridyl)-imidazol-4-carbonsäure-ethylester:
   durch Umsetzung von Azadien 1 mit 2-Aminopyridin; Schmelzpunkt 118 - 119 °C (Acetonitril).
c) 4-(3-Ethyl-1,2,4-oxadiazol-5-yl)-1-(2-pyridyl)-imidazol:
   durch Umsetzung von Azadien 2 mit 2-Aminopyridin; Schmelzpunkt 162 - 163 °C (Ethanol).
d) 4-(3-Ethyl-1,2,4-oxadiazol-5-yl)-1-(3-pyridyl)-imidazol:
   durch Umsetzung von Azadien 2 mit 3-Aminopyridin; Schmelzpunkt 152 - 153 °C (Ethanol).
e) 4-(3-Ethyl-1,2,4-oxadiazol-5-yl)-5-methyl-1-(3-pyridyl)-imidazol:
   durch Umsetzung von Azadien 3 mit 3-Aminopyridin; Schmelzpunkt 127 - 128 °C (Ethanol).
f) 4-(3-Ethyl-1,2,4-oxadiazol-5-yl)-5-methoxymethyl-1-(3-pyridyl)-imidazol:
   durch Umsetzung von Azadien 4 mit 3-Aminopyridin; Schmelpunkt 70 - 72 °C (Diisopropylether).

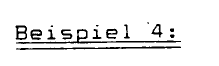

### 1-(3-Pyridyl)-imidazol-4-carbonsäure-isopropylester:

Eine Lösung von 500 mg der unter Beispiel 3a) hergestellten Verbindung in 30 ml wasserfreiem Isopropanol wird unter Stickstoff mit 0,34 ml Titanisopropylat versetzt und vier Stunden refluxiert. Danach werden noch mehrmals Portionen von je 0,04 ml Titanisopropylat zugegeben , bis auf dem Dünnschichtchromatogramm (Hexan/Aceton = 1:1) kein Ausgangsmaterial mehr zu erkenen ist. Zur Aufarbeitung wird das Reaktionsgemisch eingeengt, der Rückstand mit Essigester aufgenommen und mit wenig 2N HCl ausgeschüttelt. Danach wird mit Natriumbicarbonatlösung neutralisiert und mehrmals mit Essigester nachextrahiert. Die organische Phase wird mit Wasser gewaschen, über Natriumsulfat getrocknet und eingeengt. Aus dem Rückstand erhält man durch Chromatographie an Kieselgel mit Hexan/Aceton (6:4) 320 mg (60 %) der Titelverbindung mit einem Schmelzpunkt von 130 - 131 °C (Toluol).

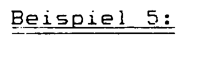

### 1-(3-Pyridyl)-imidazol-4-carbonsäure:

700 mg der unter Beispiel 4 hergestellten Verbindung werden mit 1,6 ml 2N NaOH versetzt und 5 Minuten bei Raumtemperatur gerührt. Zur Aufarbeitung werden 1,6 ml 2N HCl zugegeben , die gebildeten Kristalle abgesaugt, gründlich mit Wasser nachgewaschen und getrocknet. Auf diese Weise erhält man 540 mg (89 %) der Titelverbindung mit einem Schmelzpunkt von 265 - 266 °C (Zers.).

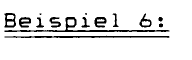

### 1-(3-Pyridyl)-imidazol-4-carbonsäure-tert.-butylester:

Eine Lösung von 230 mg der unter Beispiel 5 beschriebenen Verbindung in 10 ml wasserfreiem Dimethylformamid wird unter Schutzgas mit 2 ml Dimethylformamid-di-tert.-butylacetal versetzt und 2 ¹/₂ Stunden refluxiert. Zur Aufarbeitung wird das Reaktionsgemisch eingeengt , der Rückstand mit Toluol aufgeschlämmt und filtriert. Nach Einengen des Filtrates erhält man durch Chromatographie des Rohpoduktes an Kieselgel mit Toluol/Ethanol (95:5) 90 mg (30 %) der Titelverbindung mit einem Schmelzpunkt von 81 - 82 °C (Toluol). 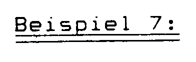
a) 1-(5-Chlor-2-pyridyl)-imidazol-4-carbonsäure-ethylester:
   Zu einer Lösung von 1,4 g Azadien 1 in 5 ml Eisessig werden unter Kühlung 643 mg 2-Amino-5-chlor-pyridin gegeben. Anschließend wird 16 Stunden unter Stickstoff bei Raumtemperatur und 3 Stunden bei 100 °C (Badtemperatur) gerührt. Zur Aufarbeitung wird das Reaktionsgemisch auf Eis/Wasser gegossen und mit Kaliumcarbonatlösung alkalisch gemacht. Der gebildete Niederschlag wird abgesaugt, grundlich mit Wasser nachgewaschen und getrocknet. Durch Umkristallisation aus Ethanol erhält man 658 mg (52 %) der Titelverbindung mit einem Schmelzpunkt von 163 - 164 °C.
   Analog Beispiel 7a) werden erhalten:
b) 1-(5-Chlor-2-pyridyl)-4-(3-ethyl-1,2,4-oxadiazol-5-yl)-imidazol:
   durch Umsetzung von Azadien 2 mit 2-Amino-5-chlor-pyridin; Schmelzpunkt 224 - 226 °C (Ethanol).
c) 1-(5-Nitro-2-pyridyl)-imidazol-4-carbonsäure-ethylester:
   durch Umsetzung von Azadien 1 mit 2-Amino-5-nitro-pyridin; Schmelzpunkt 212 - 214 °C (Ethanol).
d) 4-(3-Ethyl-1,2,4-oxadiazol-5-yl)-1-(5-nitro-2-pyridyl)-imidazol:
   durch Umsetzung von Azadien 2 mit 2-Amino-5-nitro-pyridin; Schmelzpunkt 228 - 229 °C (Ethanol).
e) 1-(3,5-Dichlor-2-pyridyl)-imidazol-4-carbonsäure-ethylester:
   durch Umsetzung von Azadien 1 mit 2-Amino-3,5-dichlor-pyridin; Schmelzpunkt 114 - 115 °C (Ethanol).
f) 1-(3,5-Dichlor-2-pyridyl)-4-(3-ethyl-1,2,4-oxadiazol-5-yl)-imidazol:
   durch Umsetzung von Azadien 2 mit 2-Amino-3,5-dichlor-pyridin; Schmelzpunkt 132 - 133 °C (Ethanol).
g) 1-(2-Pyrazinyl)-imidazol-4-carbonsäure-ethylester:
   durch Umsetzung von Azadien 1 mit 2-Amino-pyrazin; Schmelzpunkt 128 - 129 °C (Isopropanol).
h) 4-(3-Ethyl-1,2,4-oxadiazol-5-yl)-1-(2-pyrazinyl)-imidazol:
   durch Umsetzung von Azadien 2 mit 2-Aminopyrazin; Schmelzpunkt 166 - 167 °C (Ethanol).
i) 1-(1,2,4-Triazin-3-yl)-imidazol-4-carbonsäure-ethylester:
   durch Umsetzung von Azadien 1 mit 3-Amino-1,2,4-triazin; Schmelzpunkt 193 - 194 °C (Ethanol).
j) 4-(3-Ethyl-1,2,4-oxadiazol-5-yl)-1-(1,2,4-triazin-3-yl)-imidazol:
   durch Umsetzung von Azadien 2 mit 3-Amino-1,2,4-triazin; Schmelzpunkt 181 - 182 °C (Ethanol).
k) 4-(3-Ethyl-1,2,4-oxadiazol-5-yl)-1-(5-isopropoxy-2-pyrimidinyl)-imidazol:
   durch Umsetzung von Azadien 2 mit 2-Amino-5-isopropoxy-pyrimidin; Schmelzpunkt 156 - 157 °C (Diisopropylether).

## Patentansprüche

1. N-Hetaryl-imidazolderivate der allgemeinen Formel I 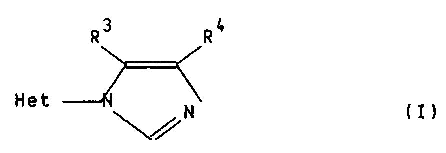 worin
Het ein mono- oder bicyclischer 5-10 gliedriger Heteroaromat mit 1-3 Schwefel-, Sauerstoff- und/oder Stickstoffatomen bedeutet, der ein bis zweifach substituiert sein kann mit Halogen, einem geradkettigen verzweigten, cyclischen, gesättigten oder ungesättigten C₁₋₆-Kohlen-wasserstoffrest, Nitro, Cyano, Thiocyanat, Azid, C₁₋₅-Alkanoyl, C₁₋₄-Alkoxycarbonyl, Carboxyl, einer gegebenenfalls mit C₁₋₄-Alkyl oder C₂₋₅-Acyl mono- oder disubstituierte Amino- oder Amidgruppe, C₁₋₄-Alkoxy, C₁₋₆-Alkylthioalkyl oder einem Sulfoxid-, Sulfonyl-oder Sulfonylaminorest,
R³ Wasserstoff, eine gerade oder verzweigte C₁₋₆-Alkylgruppe oder eine C₁₋₆-Alkoxyalkylgruppe und
R⁴ -COOR⁵, -CONR⁶R⁷, -CN, 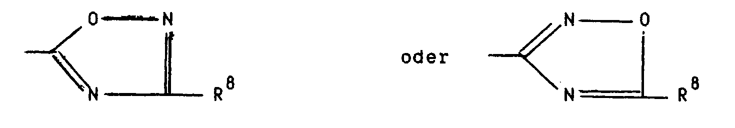
darstellt, mit R⁵ in der Bedeutung von Wasserstoff, einer geraden oder verzweigten C₁₋₆-Alkylgruppe, R⁶ und R⁷ gleich oder verschieden sind und Wasserstoff oder eine gerade, verzweigte oder cyclische Alkylgruppe mit bis zu 7 Kohlenstoffatomen darstellen oder gemeinsam mit dem Stickstoffatom einen gegebenenfalls mit ein bis zwei C¹⁻⁴-Alkylgruppen substituierten Pyrrolidin-, Piperidin-, Morpholin-, Piperazin- oder Thiomorpholin-Ring bedeuten und R₈ Wasserstoff oder eine gerade, verzweigte oder cyclische Alkylgruppe mit bis zu 7 Kohlenstoffatomen bedeutet.

2. Imidazolderivate der allgemeinen Formel I gemäß Anspruch 1, worin R⁴ COOR⁵, 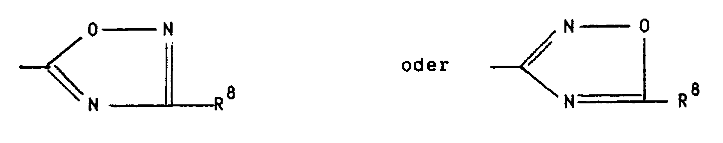 ist.

3. 1-(2-Thiazolyl)-imidazol-4-carbonsäure-ethylester
4-(3-Ethyl-1,2,4-oxadiazol-5-yl)-1-(2-thiazolyl)-imidazol
1-(5-Methyl-3-isoxazolyl)-imidazol-4-carbonsäure-ethylester
1-(5-Methyl-1,3,4-thiadiazol-2-yl)-imidazol-4-carbonsäure-ethylester
1-(1H-1,2,4-Triazol-3-yl)-imidazol-4-carbonsäure-ethylester
4-(3-Ethyl-1,2,4-oxadiazol-5-yl)-1-(1H-1,2,4-triazol-3-yl)-imidazol
1-(2-Benzothiazolyl)-imidazol-4-carbonsäure-ethylester
1-(2-Benzimidazolyl)-imidazol-4-carbonsäure-ethylester
4-(3-Ethyl-1,2,4-oxadiazol-5-yl)-1-(3-indolyl)-imidazol
1-(3-Pyridyl)-imidazol-4-carbonsäure-ethylester
1-(2-Pyridyl)-imidazol-4-carbonsäure-ethylester
4-(3-Ethyl-1,2,4-oxadiazol-5-yl)-1-(2-pyridyl)-imidazol
4-(3-Ethyl-1,2,4-oxadiazol-5-yl)-1-(3-pyridyl)-imidazol
4-(3-Ethyl-1,2,4-oxadiazol-5-yl)-5-methyl-1-(3-pyridyl)-imidazol
4-(3-Ethyl-1,2,4-oxadiazol-5-yl)-5-methoxymethyl-1-(3-pyridyl)-imidazol
1-(3-Pyridyl)-imidazol-4-carbonsäure-isopropylester
1-(3-Pyridyl)-imidazol-4-carbonsäure
1-(3-Pyridyl)-imidazol-4-carbonsäure-tert.-butylester
1-(5-Chlor-2-pyridyl)-imidazol-4-carbonsäure-ethylester
1-(5-Chlor-2-pyridyl)-4-(3-ethyl-1,2,4-oxadiazol-5-yl)-imidazaol
1-(5-Nitro-2-pyridyl)-imidazol-4-carbonsäure-ethylester
4-(3-Ethyl-1,2,4-oxadiazol-5-yl)-1-(5-nitro-2-pyridyl)-imidazol
1-(3,5-Dichlor-2-pyridyl)-imidazol-4-carbonsäure-ethylester
1-(3,5-Dichlor-2-pyridyl)-4-(3-ethyl-1,2,4-oxadizol-5-yl)-imidazol
1-(2-Pyrazinyl)-imidazol-4-carbonsäure-ethylester
4-(3-Ethyl-1,2,4-oxadiazol-5-yl)-1-(2-pyrazinyl)-imidazol
1-(1,2,4-Triazin-3-yl)-imidazol-4-carbonsäure-ethylester
4-(3-Ethyl-1,2,4-oxadiazol-5-yl)-1-(1,2,4-triazin-3-yl)-imidazol
4-(3-Ethyl-1,2,4-oxadiazol-5-yl)-1-(5-isopropoxy-2-pyrimidinyl)-imidazol
nach Anspruch 1.

4. Arzneimittel enthaltend 0.05 bis 10 mg pro Dosiseinheit einer Verbindung gemäß den Ansprüchen 1 - 3.

5. Verfahren zur Herstellung der Verbindungen der Formel I dadurch gekennzeichnet, daß man
a) ein Amin der allgemeinen Formel II
Het-NH₂ (II),
worin
Het die in Formel I angegebene Bedeutung hat, mit einem 2-Azadien der allgemeinen Formel III 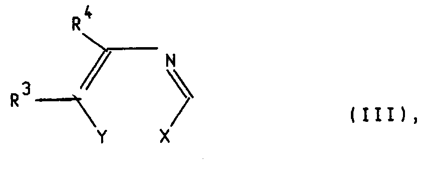 worin
R³ und R⁴ die in Formel I angegebene Bedeutung haben und
X und Y Fluchtgruppen darstellen,
in Gegenwart von einem Säureüberschuß bei Temperaturen von 0 bis 150°C umsetzt oder
b) ein Imidazolderivat der allgemeinen Formel IV 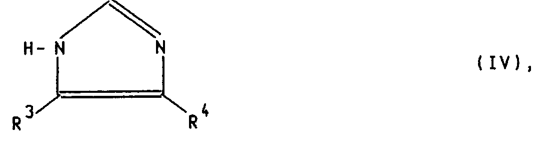 worin
R³ und R⁴ die oben genannte Bedeutung haben, mit einem Heteroaromaten der allgemeinen Formel Het-Z, worin Z eine Fluchtgruppe darstellt, N-aryliert und gegebenenfalls auftretende Isomeren trennt und anschließend gegebenenfalls in den nach a) oder b) erhaltenen Verbindungen eine im Molekül vorhandene Estergruppe umestert oder verseift, eine freie Carboxylgruppe gegebenenfallls verestert, amidiert oder mit einem Amidoxim der Formel R⁸-C(=NOH)NH₂ zum 5-Oxadiazolylderivat umsetzt und gegebenenfalls eine im Molekül vorhandene Nitrilgruppe zur Carbonylamid- oder Carboxylgruppe hydrolisiert oder über die Iminoestergruppe in die Estergruppe (COOR⁵) oder mit Hydroxylamin über das Amidoxim und anschließend mit einer Alkancarbonsäure der Formel R⁸-COOH oder einem aktivierten Derivat der Säure in das 3-Oxadiazolylderivat überführt und gegebenenfalls eine Nitro-Gruppe zur Aminogruppe reduziert und diese anschließend gegebenenfalls alkyliert, acyliert oder sulfonyliert oder über eine Diazotierung gegen Halogen, Azid, Cyano oder Thiocyanat austauscht.

6. Verfahren zur Herstellung von Arzneimitteln dadurch, daß man eine nach Anspruch 5 hergestellte Verbindung der Formel I mit einem pharmazeutisch anwendbaren Träger mischt.

## Claims

1. N-hetarylimidazole derivatives of the general formula I 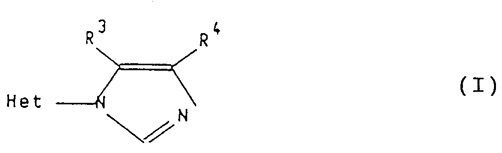 wherein
Het represents a mono- or bi-cyclic 5- to 10-membered heteroaromatic radical having from 1 to 3 sulphur, oxygen and/or nitrogen atoms which may be mono- or di-substituted by halogen; a straight-chain, branched or cyclic, saturated or unsaturated C₁₋₆-hydrocarbon radical; nitro; cyano; thiocyanate; azide; C₁₋₅-alkanoyl; C₁₋₄-alkoxycarbonyl; carboxy; an amino or amide group optionally mono- or disubstituted by C₁₋₄-alkyl or by C₂₋₅-acyl; C₁₋₄-alkoxy; C₁₋₆-alkylthioalkyl; or by a sulphoxide, sulphonyl or sulphonylamino radical;
R³ represents hydrogen, a straight-chain or branched C₁₋₆-alkyl group or a C₁₋₆-alkoxyalkyl group and
R⁴ represents -COOR⁵, -CONR⁶R⁷, -CN, 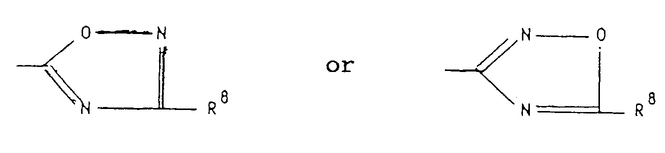 wherein R⁵ represents hydrogen or a straight-chain or branched C₁₋₆-alkyl group, R⁶ and R⁷ are the same or different and represent hydrogen or a straight-chain, branched or cyclic alkyl group having up to 7 carbon atoms, or they represent, together with the nitrogen atom, a pyrrolidine, piperidine, morpholine, piperazine or thiomorpholine ring that is optionally substituted by one or two C₁₋₄-alkyl groups, and R⁸ represents hydrogen or a straight-chain, branched or cyclic alkyl group having up to 7 carbon atoms.

2. Imidazole derivatives of the general formula I according to claim 1, wherein R⁴ is COOR⁵,

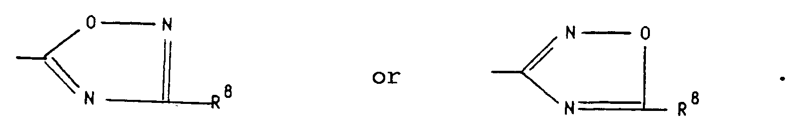

3. 1-(2-thiazolyl)-imidasole-4-carboxylic acid ethyl ester
4-(3-ethyl-1,2,4-oxadiazol-5-yl)-1-(2-thiazolyl)-imidazole
1-(5-methyl-3-isoxazolyl)-imidazole-4-carboxylic acid ethyl ester
1-(5-methyl-1,3,4-thiadiazol-2-yl)-imidazole-4-carboxylic acid ethyl ester
1-(1H-1,2,4-triazol-3-yl)-imidazole-4-carboxylic acid ethyl ester
4-(3-ethyl-1,2,4-oxadiazol-5-yl)-1-(1H-1,2,4-triazol-3-yl)-imidazole
1-(2-benzothiazolyl)-imidazole-4-carboxylic acid ethyl ester
1-(2-benzimidazolyl)-imidazole-4-carboxylic acid ethyl ester
4-(3-ethyl-1,2,4-oxadiazol-5-yl)-1-(3-indolyl)-imidazole
1-(3-pyridyl)-imidazole-4-carboxylic acid ethyl ester
1-(2-pyridyl)-imidazole-4-carboxylic acid ethyl ester
4-(3-ethyl-1,2,4-oxadiazol-5-yl)-1-(2-pyridyl)-imidazole
4-(3-ethyl-1,2,4-oxadiazol-5-yl)-1-(3-pyridyl)-imidazole
4-(3-ethyl-1,2,4-oxadiazol-5-yl)-5-methyl-1-(3-pyridyl)-imidazole
4-(3-ethyl-1,2,4-oxadiazol-5-yl)-5-methoxymethyl-1-(3-pyridyl)-imidazole
1-(3-pyridyl)-imidazole-4-carboxylic acid isopropyl ester
1-(3-pyridyl)-imidazole-4-carboxylic acid
1-(3-pyridyl)-imidazole-4-carboxylic acid tert-butyl ester
1-(5-chloro-2-pyridyl)-imidazole-4-carboxylic acid ethyl ester
1-(5-chloro-2-pyridyl)-4-(3-ethyl-1,2,4-oxadiazol-5-yl)-imidazole
1-(5-nitro-2-pyridyl)-imidazole-4-carboxylic acid ethyl ester
4-(3-ethyl-1,2,4-oxadiazol-5-yl)-1-(5-nitro-2-pyridyl)-imidazole
1-(3,5-dichloro-2-pyridyl)-imidazole-4-carboxylic acid ethyl ester
1-(3,5-dichloro-2-pyridyl)-4-(3-ethyl-1,2,4-oxadiazol-5-yl)-imidazole
1-(2-pyrazinyl)-imidazole-4-carboxylic acid ethyl ester
4-(3-ethyl-1,2,4-oxadiazol-5-yl)-1-(2-pyrazinyl)-imidazole
1-(1,2,4-triazin-3-yl)-imidazole-4-carboxylic acid ethyl ester
4-(3-ethyl-1,2,4-oxadiazol-5-yl)-1-(1,2,4-triazin-3-yl)-imidazole
4-(3-ethyl-1,2,4-oxadiazol-5-yl)-1-(5-isopropoxy-2-pyrimidinyl)-imidazole
according to claim 1.

4. Medicament containing from 0.05 to 10 mg per unit dose of a compound according to claims 1 to 3.

5. Process for the preparation of the compounds of formula I, characterised in that
a) an amine of the general formula II
Het-NH₂ (II),
wherein
Het is as defined in formula I, is reacted with a 2-azadiene of the general formula III 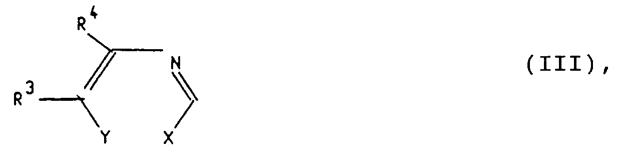 wherein
R³ and R⁴ are as defined in formula I and
X and Y are leaving groups,
in the presence of an excess of acid at temperatures of from 0 to 150°C or
b) an imidazole derivative of the general formula IV 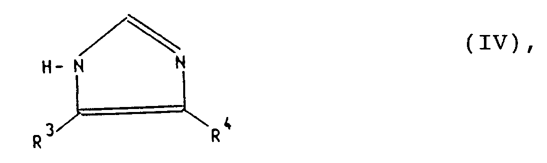 wherein
R³ and R⁴ are as defined above, is N-arylated with a heteroaromatic radical of the general formula Het-Z, wherein Z is a leaving group, and, optionally, resulting isomers are separated and then, optionally, in the compounds obtained according to a) or b), an ester group present in the molecule is transesterified or hydrolysed, a free carboxy group is optionally esterified, amidated or reacted with an amidoxime of the formula R⁸-C(=NOH)NH₂ to form the 5-oxadiazolyl derivative and, optionally, a nitrile group present in the molecule is hydrolysed to form the carbonyl amide or carboxy group or is converted by way of the imino ester group into the ester group (COOR⁵) or with hydroxylamine by way of the amidoxime and subsequently with an alkanecarboxylic acid of the formula R⁸-COOH or an activated derivative of the acid into the 3-oxadiazolyl derivative and, optionally, a nitro group is reduced to the amino group and the latter is then optionally alkylated, acylated or sulphonylated or is replaced by halogen, azide, cyano or thiocyanate by diazotisation.

6. Process for the preparation of medicaments by mixing a compound of formula I prepared according to claim 5 with a pharmaceutically acceptable carrier.

## Revendications

1. Dérivés de N-hétaryl-imidazole répondant à la formule générale I 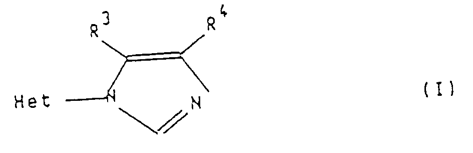 dans laquelle
Het signifie un hétéroaromatique mono- ou bicyclique comportant de 5 à 10 chaînons avec 1 à 3 atomes de soufre, d'oxygène et/ou d'azote, qui peut être mono- ou bisubstitué par un halogène, par un radical hydrocarboné en C₁₋₆ linéaire, ramifié, cyclique, saturé ou non saturé, par un nitro, par un cyano, par un thiocyanate, par un azide, par un alcanoyle en C₁₋₅, par un alcoxycarbonyle en C₁₋₄, par un carboxyle, un groupe amino ou amide éventuellement mono- ou bisubstitué par un alkyle en C₁₋₄ ou un acyle en C₂₋₅, par un alcoxy en C₁₋₄, par un alkylthioalkyle en C₁₋₆ ou par un radical sulfoxyde, sulfonyle ou sulfonylamino,
R³ représente l'hydrogène, un groupe alkyle en C₁₋₆ linéaire ou ramifié ou un groupe alcoxyalkyle en C₁₋₆ et
R⁴ représente -COOR⁵, -CONR⁶R⁷, -CN, 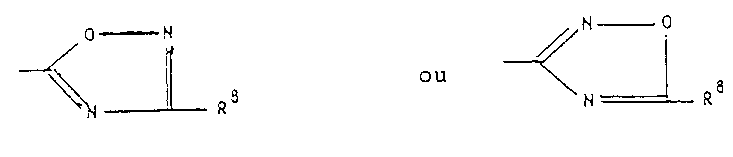 avec R⁵ signifiant l'hydrogène, un groupe alkyle en C₁₋₆ linéaire ou ramifié, R⁶ et R⁷ sont identiques ou différents et représentent l'hydrogène ou un groupe alkyle linéaire, ramifié ou cyclique avec un nombre d'atomes de carbone allant jusqu'à 7 ou ensemble avec l'atome d'azote signifient un cycle pyrrolidine, pipéridine, morpholine, pipérazine ou thiomorpholine éventuellement substitué par un à deux groupes alkyles en C₁₋₄ et R⁸ signifie l'hydrogène ou un groupe alkyle linéaire, ramifié ou cyclique avec un nombre d'atomes de carbone allant jusqu'à 7.

2. Dérivés d'imidazoles répondant à la formule générale I selon la revendication 1, où R⁴ est COOR⁵,

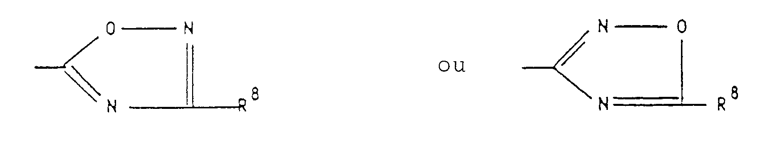

3. le 1-(2-thiazolyl)-imidazol-4-carboxylate d'éthyle ;
le 4-(3-éthyl-1,2,4-oxadiazol-5-yl)-1-(2-thiazolyl)-imidazole ;
le 1-(5-méthyl-3-isoxazolyl)-imidazol-4-carboxylate d'éthyle ;
le 1-(5-méthyl-1,3,4-thiadiazol-2-yl)-imidazol-4-carboxylate d'éthyle ;
le 1-(1H-1,2,4-triazol-3-yl)-imidazol-4-carboxylate d'éthyle ;
le 4-(3-éthyl-1,2,4-oxadiazol-5-yl)-1-(1H-1,2,4-triazol-3-yl)-imidazole ;
le 1-(2-benzothiazolyl)-imidazol-4-carboxylate d'éthyle ;
le 1-(2-benzimidazolyl)-imidazol-4-carboxylate d'éthyle ;
le 4-(3-éthyl-1,2,4-oxadiazol-5-yl)-1-(3-indolyl)-imidazole ;
le 1-(3-pyridyl)-imidazol-4-carboxylate d'éthyle ;
le 1-(2-pyridyl)-imidazol-4-carboxylate d'éthyle ;
le 4-(3-éthyl-1,2,4-oxadiazol-5-yl)-1-(2-pyridyl)-imidazole ;
le 4-(3-éthyl-1,2,4-oxadiazol-5-yl)-1-(3-pyridyl)-imidazole ;
le 4-(3-éthyl-1,2,4-oxadiazol-5-yl)-5-méthyl-1-(3 pyridyl)-imidazole ;
le 4-(3-éthyl-1,2,4-oxadiazol-5-yl)-5-méthoxyméthyl-1-(3-pyridyl)-imidazole ;
le 1-(3-pyridyl)-imidazol-4-carboxylate d'isopropyle ;
l'acide 1-(3-pyridyl)-imidazol-4-carboxylique ;
le 1-(3-pyridyl)-imidazol-4-carboxylate de tert.-butyle ;
le 1-(5-chloro-2-pyridyl)-imidazol-4-carboxylate d'éthyle ;
le 1-(5-chloro-2-pyridyl)-4-(3-éthyl-1,2,4-oxadiazol-5-yl)-imidazole ;
le 1-(5-nitro-2-pyridyl)-imidazol-4-carboxylate d'éthyle ;
le 4-(3-éthyl-1,2,4-oxadiazol-5-yl)-1-(5-nitro-2-pyridyl)-imidazole ;
le 1-(3,5-dichloro-2-pyridyl)-imidazol-4-carboxylate d'éthyle ;
le 1-(3,5-dichloro-2-pyridyl)-4-(3-éthyl-1,2,4-oxadiazol-5-yl)-imidazole ;
le 1-(2-pyrazinyl)-imidazol-4-carboxylate d'éthyle ;
le 4-(3-éthyl-1,2,4-oxadiazol-5-yl)-1-(2-pyrazinyl)-imidazole ;
le 1-(1,2,4-triazin-3-yl)-imidazol-4-carboxylate d'éthyle ;
le 4-(3-éthyl-1,2,4-oxadiazol-5-yl)-1-(1,2,4-triazin-3-yl)-imidazole ;
le 4-(3-éthyl-1,2,4-oxadiazol-5-yl)-1-(5-isopropoxy-2-pyrimidinyl)-imidazole ;
selon la revendication 1.

4. Médicament contenant de 0,05 à 10 mg par dose unitaire d'un composé selon les revendications 1-3.

5. Procédé pour la préparation des composés répondant à la formule I, caractérisé en ce qu'on fait réagir
a) une amine de formule générale II
Het-NH₂ (II)
dans laquelle
Het a la signification donnée dans la formule I, avec un 2-azadiène de formule générale III 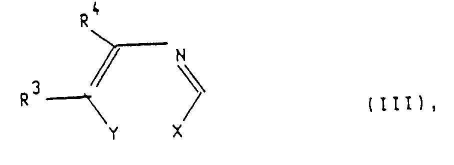 dans laquelle
R³ et R⁴ ont la signification donnée dans la formule I et
X et Y représentent des groupes transitoires,
en présence d'un excès d'acide à des températures de 0 à 150°C ou
b) un dérivé d'imidazole répondant à la formule générale IV 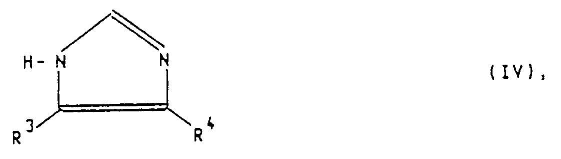 dans laquelle
R³ et R⁴ ont la signification donnée ci-dessus, est N-arylé avec un hétéroaromatique de formule générale Het-Z où Z représente un groupe transitoire et les isomères éventuellement formés sont séparés et ensuite éventuellement un groupe ester présent dans la molécule des composés obtenus selon a) ou b) est transestérifié ou saponifié, un groupe carboxyle libre est éventuellement estérifié, amidifié ou transformé à l'aide d'une amidoxime de formule R⁸-C(=NOH)NH₂ en le dérivé 5-oxadiazolylique et éventuellement un groupe nitrile présent dans la molécule est hydrolysé en groupe carbonylamide ou carboxyle ou est transformé par intermédiaire du groupe imino-ester en le groupe ester (COOR⁵), ou est transformé par l'hydroxylamine par l'intermédiaire de l'amidoxime et ensuite par un acide alcanecarboxylique de formule R⁸-COOH ou un dérivé activé de l'acide, en la dérivé 3-oxadiazolylique et un groupe nitro est éventuellement réduit en groupe amino et celui-ci est ensuite éventuellement alkylé, acylé ou sulfonylé ou remplacé par un halogène, un azide, un cyano ou un thiocyanate par l'intermédiaire d'une diazotation.

6. Procédé pour la préparation de médicaments (caractérisé) en ce qu'on mélange un composé de formule I préparé selon la revendication 5, avec un véhicule pharmaceutiquement utilisable.
